# EUROPEAN PATENT APPLICATION

(11) **EP 4 538 686 A1**
(43) Date of publication of application: **16.04.2025**
(21) Application number: 23819234.8
(22) Date of filing: 08.06.2023
(51) Int. Cl.: G01N 23/046

(54) **FLEXIBLE CT DETECTOR AND STATIC CT SYSTEM USING SAME**

(30) Priority: 08.06.2022 CN 202210648188
(71) Applicant: Nanovision Technology (Beijing) Co., Ltd., Beijing 100094 (CN)
(72) Inventor: ZHANG, Chao, Beijing 100094 (CN); CUI, Zhili, Beijing 100094 (CN); LI, Yunxiang, Beijing 100094 (CN)
(74) Representative: Wang, Bo
(86) International application number: PCT/CN2023/099243
(87) International publication number: WO 2023/237070

(57) **Abstract**

A flexible CT detector and a static CT system using same. The flexible CT detector comprises: a flexible circuit board (1), which is used for being attached onto the inner side of a CT frame (10) and has a signal reading portion; and a flexible conversion portion (2), which is attached onto the inner side of the flexible circuit board (1) and is used for absorbing an X-ray and converting same into a set signal, wherein the signal reading portion is used for receiving the set signal and outputting continuous and uninterrupted projection images. In the flexible CT detector, a large-area flexible circuit is used as a substrate of the detector, such that pixels of the whole ring of the detector are arranged closely, and the problems of splicing seams and projection information loss that are caused by the assembly of a traditional rigid detector are greatly reduced; and in an ideal situation, there is only one splicing seam at an end-to-end joint of the whole ring of the detector, such that the difficulty of algorithm correction is greatly reduced.

## Description

### BACKGROUND

### Technical Field

The present invention relates to a flexible CT detector, and further relates to a static CT system using the flexible CT detector, and belongs to the field of computed tomography technologies.

### Related Art

CT is an abbreviation for computed tomography. The CT uses an accurately collimated X-ray and the like to perform cross-sectional scanning around a part of a human body together with a CT detector with extremely high sensitivity, has characteristics such as short scanning time and clear images, and can be used in many fields such as medical treatment and security check. A static CT system including a plurality of radiation sources and a full-ring detector can break through a physical rotation speed limit of a conventional mechanical rotation scanning manner to obtain a higher scanning speed, and has become a hotspot for current academic research and industry transformation.

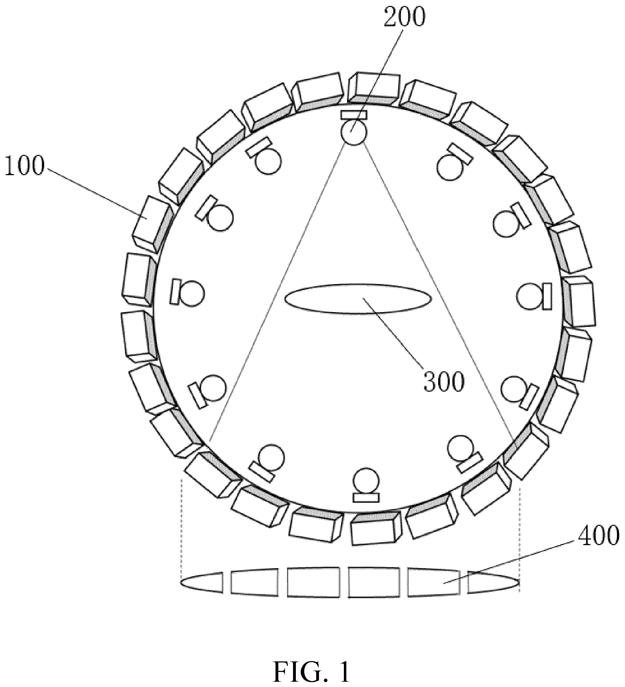

As shown in FIG. 1, to achieve a large-range coverage satisfying clinically relevant diagnostics, a typical static CT system is generally formed by splicing a plurality of photon counting detectors or integrating detectors 100, and each detector module is further formed by splicing a plurality of detector units. For the static CT system, there may be splicing joints of tens or even hundreds of detectors for the full-ring detector, and a size of each splicing joint is affected by dimensional accuracy and assembly accuracy of the detectors.

However, an electronic readout system based on a silicon-based photodiode or a complementary metal-oxide semiconductor (CMOS) inevitably has a problem of projection information loss in a splicing joint part. After a radiation source 200 emits an X-ray to a to-be-detected object 300, a presented exposure projection 400 is in a discontinuous form. In addition, to obtain higher spatial resolution and a larger scanning range, the CT detector continuously pursues a smaller pixel size and a larger quantity of rows. For splicing joints of the same physical size, a smaller pixel size of the detector indicates more lost information that needs to be offset by an algorithm, which severely affects further improvement in spatial resolution of a CT image.

### SUMMARY

A primary technical problem to be resolved by the present invention is to provide a flexible CT detector, to reduce problems of splicing joints and projection information loss caused by assembly of the detector.

Another technical problem to be resolved by the present invention is to provide a static CT system using the foregoing flexible CT detector.

To achieve the foregoing objective, the present invention uses the following technical solutions:
According to a first aspect of the embodiments of the present invention, a flexible CT detector is provided, including:
a flexible circuit board, configured to be mounted on an inner side of a CT frame and including a signal reading portion; and
a flexible conversion portion, mounted on an inner side of the flexible circuit board, to absorb an X-ray and convert the X-ray into a set signal, where the signal reading portion is configured to receive the set signal and output a continuous projected image.

Preferably, the signal reading portion includes an electronic readout circuit and a plurality of columns of pixelated electrodes, wherein
the electronic readout circuit is depicted in the flexible circuit board, the plurality of columns of pixelated electrodes are arranged on a surface of the flexible circuit board in a length direction of the flexible circuit board, and the plurality of columns of pixelated electrodes are connected to the electronic readout circuit; and
the flexible conversion portion includes an X-ray absorption and conversion layer for absorbing an X-ray and converting the X-ray into an electrical signal.

Preferably, the flexible conversion portion further includes an electron transport layer and a hole transport layer, wherein
the electron transport layer is mounted between an outer side of the X-ray absorption and conversion layer and the inner side of the flexible circuit board, to transport an electron and block a hole; and
the hole transport layer is mounted on an inner side of the X-ray absorption and conversion layer to transport a hole and block an electron, where a position of the electron transport layer is interchangeable with a position of the hole transport layer.

Preferably, the flexible conversion portion further includes a common electrode layer and a power supply, wherein
the common electrode layer is mounted on an inner side of the hole transport layer, the common electrode layer is connected to one electrode of the power supply, and the other electrode of the power supply is connected to the flexible circuit board, to form an electric field for driving movement of an electron hole pair.

Preferably, the signal reading portion includes a visible light readout circuit and a plurality of columns of pixelated photosensitive units, wherein
the visible light readout circuit is depicted in the flexible circuit board, the plurality of columns of pixelated photosensitive units are arranged on a surface of the flexible circuit board in a length direction of the flexible circuit board, and the plurality of columns of pixelated photosensitive units are connected to the visible light readout circuit; and
the flexible conversion portion is a flexible scintillator film, configured to absorb an X-ray and convert the X-ray into an optical signal.

Preferably, the flexible circuit board includes a plurality of flexible circuit sub-boards, and the plurality of flexible circuit sub-boards are sequentially spliced end to end, to be mounted on the inner side of the CT frame.

Preferably, lengths of the plurality of flexible circuit sub-boards are all the same, all different, or partially the same.

Preferably, the flexible CT detector further includes a waterproof layer, where the waterproof layer is wrapped around outer sides of the flexible circuit board and the flexible conversion portion.

According to a second aspect of the embodiments of the present invention, a static CT system is provided, including:
a CT frame;
the foregoing flexible CT detector, mounted on an inner side of the CT frame; and
at least one X-ray source, evenly distributed on an inner side of the flexible CT detector, to emit an X-ray toward a to-be-scanned object.

Preferably, a control circuit is arranged on the CT frame, and the control circuit is connected to the flexible circuit board, to control the signal reading portion to perform signal reading.

Compared with the related art, according to the flexible CT detector and the static CT system thereof provided in the present invention, by using a large-area flexible circuit as a substrate of the detector, pixels of a full-ring detector are closely arranged, which greatly reduces problems of splicing joints and projection information loss caused by assembly of a conventional rigid detector. Ideally, the full-ring detector has only one splicing joint at aa head-to-tail junction, which greatly reduces difficulty of algorithm correction.

### BRIEF DESCRIPTION OF THE DRAWINGS

FIG. 1 is a schematic diagram of a splicing structure of a typical static CT system;
FIG. 2 is a schematic structural diagram of a flexible CT detector according to a first embodiment of the present invention;
FIG. 3 is a schematic structural diagram of a flexible CT detector according to a second embodiment of the present invention;
FIG. 4 is a schematic structural diagram of a flexible CT detector according to a third embodiment of the present invention; and
FIG. 5 is a schematic structural diagram of a static CT system according to a fourth embodiment of the present invention.

### DETAILED DESCRIPTION

The technical content of the present invention is described in detail below with reference to the accompanying drawings and specific embodiments.

### <First Embodiment>

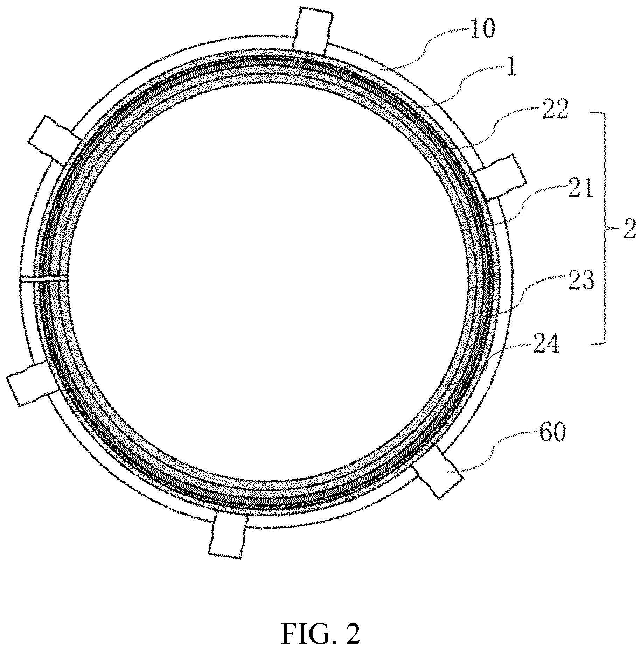

FIG. 2 shows a flexible CT detector according to a first embodiment of the present invention. The flexible CT detector includes at least: a flexible circuit board 1 and a flexible conversion portion 2.

The flexible circuit board 1 is configured to be mounted on an inner side of a CT frame 10, and includes a signal reading portion. The flexible conversion portion 2 is mounted on an inner side of the flexible circuit board 1 (that is, a side A in FIG. 2), to absorb an X-ray and convert the X-ray into a set signal, and the signal reading portion is configured to receive the set signal and output a continuous projected image.

In this embodiment, because the flexible circuit board 1 may be entirely mounted on the inner side of the CT frame 10, a splicing joint between different modules can be greatly reduced by adjusting a size of the flexible circuit board 1 to match that of the CT frame 10. Ideally, there is only one head-to-tail abutting gap, and the abutting gap is smaller than the splicing joint between different modules. Therefore, problems of projection information loss and image discontinuity caused by the splicing joint between different modules in a detector of a static CT system can be avoided or alleviated, and imaging quality of the static CT system can be improved.

In the foregoing embodiment, the flexible circuit board 1 is optionally a sensor panel such as a TFT or an IGZO. In addition, to cause the size of the flexible circuit board 1 to match that of the CT frame 10, the flexible circuit board 1 should have a width not greater than that of the CT frame 10 and a length equivalent to a perimeter (an inner perimeter) of the CT frame 10, so that the flexible circuit board 1 can be closely attached to the inner side of the CT frame 10.

In addition, after the flexible circuit board 1 is closely attached to the inner side of the CT frame 10, the head and the tail of the flexible circuit board 1 need to be closely attached and fixed relative to each other. A relative fixing manner may be a bolt, a carbon fiber cover plate, gluing, or the like, to ensure mounting stability of the flexible circuit board 1.

In the foregoing embodiment, the signal reading portion includes an electronic readout circuit and a plurality of columns of pixelated electrodes. The electronic readout circuit is depicted in the flexible circuit board 1. The plurality of columns of pixelated electrodes are arranged on a surface of the flexible circuit board 1 in a length direction of the flexible circuit board 1, and the plurality of columns of pixelated electrodes are connected to the electronic readout circuit, to receive an electronic signal.

Correspondingly, the flexible conversion portion 2 includes an X-ray absorption and conversion layer 21, an electron transport layer 22, a hole transport layer 23, a common electrode layer 24, and a power supply. The X-ray absorption and conversion layer 21 is located on the inner side of the flexible circuit board 1 to absorb an X-ray and convert the X-ray into an electrical signal. The electron transport layer 22 is mounted between an outer side of the X-ray absorption and conversion layer 21 and the inner side of the flexible circuit board 1, to transport an electron and block a hole. The hole transport layer 23 is mounted on an inner side of the X-ray absorption and conversion layer to transport a hole and block an electron. The common electrode layer 24 is mounted on an inner side of the hole transport layer 23, the common electrode layer 24 is connected to one electrode of the power supply, and the other electrode of the power supply is connected to the flexible circuit board 1, to form an electric field for driving movement of an electron hole pair.

In the foregoing embodiment, it may be understood that a position of the electron transport layer 22 is interchangeable with a position of the hole transport layer 23. Correspondingly, a direction of the electric field for driving movement of the electron hole pair also needs to be flipped, and may be specifically adaptively selected according to actual needs.

It may be understood that, in the foregoing embodiment, the flexible circuit board 1 collaborates with the flexible conversion portion 2 to jointly form a photon counting detector. During specific use, after the radiation source emits an X-ray, the X-ray is absorbed and converted into an electrical signal (that is, an electron hole pair) by the X-ray absorption and conversion layer 21. Then, the electron hole pair is driven to move by using an electric field formed by the common electrode layer 24 in collaboration with the power supply and the flexible circuit board 1. During movement of the electron hole pair, arrangement of the electron transport layer 22 and the hole transport layer 23 can effectively improve movement efficiency of the electron hole pair. Finally, the plurality of columns of pixelated electrodes on the flexible circuit board 1 are used to receive the electrical signal, and the electrical signal is read out through the electronic readout circuit to output a photon counting image.

In the foregoing embodiment, the flexible CT detector further includes a waterproof layer, and the waterproof layer is wrapped around outer sides of the flexible circuit board 1 and the flexible conversion portion 2. The waterproof layer includes, but not limited to, a PI film, a PET film, an evaporated parylene film, or a composite coating. The composite coating is formed by the foregoing PI film, PET film, or evaporated parylene film in collaboration with a dense inorganic waterproof film made of SiO₂, TiO₂, Al₂O₃, or the like. The use of the waterproof layer can improve waterproof and moisture-proof performance of the entire flexible CT detector, thereby improving use safety.

In summary, according to the flexible CT detector provided in the first embodiment of the present invention, by using a large-area flexible circuit as a substrate of the detector, pixels of a full-ring detector are closely arranged, which greatly reduces problems of splicing joints and projection information loss caused by assembly of a conventional rigid detector. Ideally, the full-ring detector has only one splicing joint at aa head-to-tail junction, which greatly reduces difficulty of algorithm correction. In addition, the flexible conversion portion 2 for optical-to-electrical conversion is in a structural form of a composite film, which improves efficiency of the optical-to-electrical conversion, thereby helping improve the definition of the photon counting image.

### <Second Embodiment>

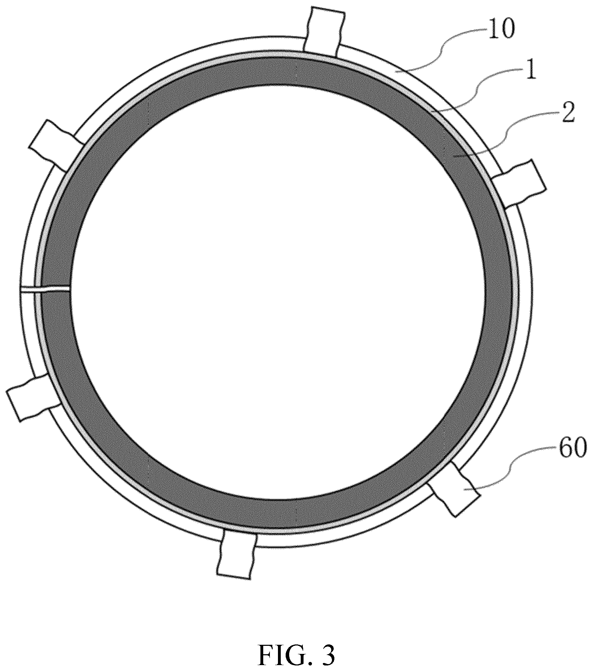

FIG. 3 shows a flexible CT detector according to a second embodiment of the present invention. The flexible CT detector includes at least: a flexible circuit board 1 and a flexible conversion portion 2. A difference between this embodiment and the first embodiment is that in this embodiment, a type of the flexible CT detector is different from that in the first embodiment.

Specifically, in this embodiment, the signal reading portion includes a visible light readout circuit and a plurality of columns of pixelated photosensitive units. The visible light readout circuit is depicted in the flexible circuit board 1, the plurality of columns of pixelated photosensitive units are arranged on a surface of the flexible circuit board 1 in a length direction of the flexible circuit board 1, and the plurality of columns of pixelated photosensitive units are connected to the visible light readout circuit to receive an optical signal. Correspondingly, the flexible conversion portion 2 is a flexible scintillator film, to absorb an X-ray and convert the X-ray into the optical signal.

Therefore, in this embodiment, a detector formed by the flexible circuit board 1 in collaboration with the flexible conversion portion 2 is an integrating detector. During specific use, after the radiation source emits an X-ray, the X-ray is converted into an optical signal by using the flexible conversion portion 2 formed by the flexible scintillator film. Then, the optical signal is received by using the plurality of columns of pixelated photosensitive units on the flexible circuit board 1. Finally, the optical signal is read out by using the visible light readout circuit, to output an energy integrating image.

In this embodiment, all structures except for the foregoing distinguishable structure are the same as those in the first embodiment, and details are not described herein again.

### <Third Embodiment>

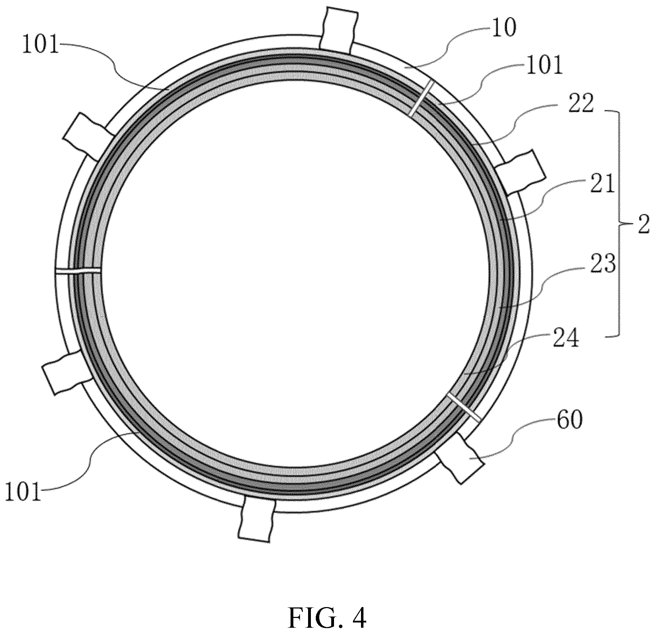

As shown in FIG. 4, based on the first embodiment, a third embodiment of the present invention provides a flexible CT detector, and a difference from the first embodiment is that the flexible circuit board 1 in this embodiment is of a multi-segment splicing structure.

Specifically, in this embodiment, the flexible circuit board 1 includes a plurality of flexible circuit sub-boards 101, and the plurality of flexible circuit sub-boards 101 are sequentially spliced end to end, to be mounted on the inner side of the CT frame. Preferably, in the flexible circuit board 1, 2 to 32 flexible circuit sub-boards 101 are connected end to end to form a full-ring detector. However, a specific quantity of flexible circuit sub-boards 101 is not limited, and may be adaptively selected according to needs.

It may be understood that, for a flexible circuit board 1 with a set length, when a quantity of flexible circuit sub-boards 101 is larger, manufacturing difficulty is lower, and a corresponding quantity of splicing joints increases as the quantity of flexible circuit sub-boards 101 increases; otherwise, the manufacturing difficulty of the flexible circuit sub-board 101 is higher, and a correspondingly quantity of splicing j oints decreases as the quantity of the flexible circuit sub-boards 101 decreases. However, compared with a conventional rigid detector, even if the flexible circuit board 1 of this application is in a form in which a plurality of flexible circuit sub-boards 101 are spliced, a size of the splicing joint itself is greatly reduced, thereby alleviating problems of projection information loss and image discontinuity.

In addition, in a variation of the present invention, lengths of the plurality of flexible circuit sub-boards 101 are all the same. In another variation, lengths of the plurality of flexible circuit sub-boards 101 are all different. In still another variation, lengths of the plurality of flexible circuit sub-boards 101 are only partially the same. The lengths may be specifically selected according to actual usage conditions to adapt to CT frames of different sizes and structures.

In this embodiment, all structures except for the foregoing distinguishable structure are the same as those in the first embodiment, and details are not described herein again.

### <Fourth Embodiment>

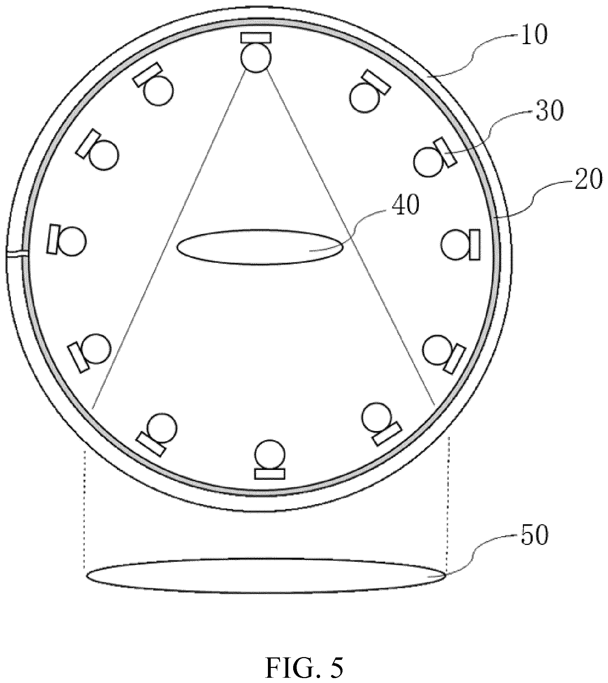

FIG. 5 shows a static CT system according to a fourth embodiment of the present invention. The static CT system includes: a CT frame 10, a flexible CT detector 20, and a plurality of X-ray sources 30.

The CT frame is configured to provide a mounting basis for the flexible CT detector 20. The flexible CT detector 20 is mounted on an inner side of the CT frame 10. The plurality of X-ray sources 30 are evenly distributed on an inner side of the flexible CT detector 20 to emit an X-ray toward a to-be-scanned object 40, and a continuous projected image 50 is formed by using the flexible CT detector 20.

In this embodiment, the CT frame 10 is in a circular ring shape. In other embodiments, the CT frame 10 may alternatively be in an elliptical shape, a square shape, or another shape. In addition, the CT frame 10 may alternatively be in a spiral shape, so that the entire flexible CT detector 20 is spirally mounted on the CT frame 10. In this way, there is no splicing joint for the entire arc-shaped detector, and it is ensured that pixels are completely continuous.

In the foregoing embodiment, a control circuit 60 is arranged on the CT frame 10, and the control circuit is connected to the flexible circuit board 1 to control the signal reading portion to perform signal reading. Therefore, CT detection can be automatically controlled by using the control circuit 60, thereby improving convenience of CT detection.

In addition, in another variation of the present invention, there may be only one radiation source 30, and there is no need to select the full-ring detector as the detector 20 as long as the detector 20 is arc-shaped (that is, a part of the flexible detector in FIG. 4). Then, the detector 20 rotates at a high speed with the radiation source 30 to form spiral CT, which can also reduce impact of a splicing joint inside the detector 20.

It should be noted that, embodiments or variations in the present invention are all described in a related manner, mutual reference may be made to identical or similar parts of the embodiments or variations. Each embodiment or variation focuses on illustrating a difference from other embodiments, but each embodiment or variation is implemented based on working principles of the flexible CT detector and the static CT system thereof. Details are not described herein again.

Compared with the related art, according to the flexible CT detector and the static CT system thereof provided in the present invention, by using a large-area flexible circuit as a substrate of the detector, pixels of a full-ring detector are closely arranged, which greatly reduces problems of splicing joints and projection information loss caused by assembly of a conventional rigid detector. Ideally, the full-ring detector has only one splicing joint at aa head-to-tail junction, which greatly reduces difficulty of algorithm correction.

The flexible CT detector and the static CT system using same provided in the present invention are described in detail above. For a person of ordinary skill in the art, any obvious modifications made to the present invention without departing from the essence of the present invention will constitute an infringement of patent rights of the present invention, and corresponding legal liabilities will be born.

## Claims

1. A flexible computed tomography (CT) detector, comprising:
a flexible circuit board, configured to be mounted on an inner side of a CT frame and comprising a signal reading portion; and
a flexible conversion portion, mounted on an inner side of the flexible circuit board, to absorb an X-ray and convert the X-ray into a set signal, wherein the signal reading portion is configured to receive the set signal and output a continuous projected image.

2. The flexible CT detector according to claim 1, wherein the signal reading portion comprises an electronic readout circuit and a plurality of columns of pixelated electrodes, wherein
the electronic readout circuit is depicted in the flexible circuit board, the plurality of columns of pixelated electrodes are arranged on a surface of the flexible circuit board in a length direction of the flexible circuit board, and the plurality of columns of pixelated electrodes are connected to the electronic readout circuit; and
the flexible conversion portion comprises an X-ray absorption and conversion layer for absorbing an X-ray and converting the X-ray into an electrical signal.

3. The flexible CT detector according to claim 2, wherein the flexible conversion portion further comprises an electron transport layer and a hole transport layer, wherein
the electron transport layer is mounted between an outer side of the X-ray absorption and conversion layer and the inner side of the flexible circuit board, to transport an electron and block a hole; and
the hole transport layer is mounted on an inner side of the X-ray absorption and conversion layer to transport a hole and block an electron, wherein a position of the electron transport layer is interchangeable with a position of the hole transport layer.

4. The flexible CT detector according to claim 3, wherein the flexible conversion portion further comprises a common electrode layer and a power supply, wherein
the common electrode layer is mounted on an inner side of the hole transport layer, the common electrode layer is connected to one electrode of the power supply, and the other electrode of the power supply is connected to the flexible circuit board, to form an electric field for driving movement of an electron hole pair.

5. The flexible CT detector according to claim 1, wherein the signal reading portion comprises a visible light readout circuit and a plurality of columns of pixelated photosensitive units, wherein
the visible light readout circuit is depicted in the flexible circuit board, the plurality of columns of pixelated photosensitive units are arranged on a surface of the flexible circuit board in a length direction of the flexible circuit board, and the plurality of columns of pixelated photosensitive units are connected to the visible light readout circuit; and
the flexible conversion portion is a flexible scintillator film, configured to absorb an X-ray and convert the X-ray into an optical signal.

6. The flexible CT detector according to claim 1, wherein the flexible circuit board comprises a plurality of flexible circuit sub-boards, and the plurality of flexible circuit sub-boards are sequentially spliced end to end, to be mounted on the inner side of the CT frame.

7. The flexible CT detector according to claim 6, wherein lengths of the plurality of flexible circuit sub-boards are all the same, all different, or partially the same.

8. The flexible CT detector according to any one of claims 1 to 7, further comprising a waterproof layer, wherein the waterproof layer is wrapped around outer sides of the flexible circuit board and the flexible conversion portion.

9. A static CT system, comprising:
a CT frame;
the flexible CT detector according to any one of claims 1 to 8, wherein the flexible CT detector is mounted on an inner side of the CT frame; and
at least one X-ray source, distributed on an inner side of the flexible CT detector, to emit an X-ray toward a to-be-scanned object.

10. The static CT system according to claim 9, wherein a control circuit is arranged on the CT frame, and the control circuit is connected to the flexible circuit board, to control the signal reading portion to perform signal reading.
